# FASCICULE DE BREVET EUROPEEN

(11) **EP 2 596 086 B1**
(45) Date de publication et mention de la délivrance du brevet: **13.06.2018**
(21) Numéro de dépôt: 11752262.3
(22) Date de dépôt: 21.07.2011
(51) Int. Cl.: C10G 53/12, C10G 31/08, C10G 19/08, C10G 21/06, C10G 19/02, C10G 53/04, B01D 53/26, B01D 53/28, C10G 33/04, C07C 7/12

(54) **PROCÉDÉ ET INSTALLATION DE DÉSHYDRATATION PAR PRODUIT DÉLIQUESCENT**
VERFAHREN UND ANLAGE FÜR DEHYDRIERUNGEN MITHILFE EINER ZERFLIESSENDEN SUBSTANZ
PROCESS AND PLANT FOR DEHYDRATION BY A DELIQUESCENT SUBSTANCE

(30) Priorité: 23.07.2010 FR 1003091
(43) Date de publication de la demande: 29.05.2013
(73) Titulaire: IFP Energies nouvelles, 92500 Rueil-Malmaison (FR); Newton's, 84300 Cavaillon (FR)
(72) Inventeur: AUGIER, Frédéric, F-69360 Saint Symphorien d'Ozon (FR); VASSIEU, Maxime, F-84300 Cavaillon (FR)
(86) Numéro de dépôt international: PCT/FR2011/000434
(87) Numéro de publication internationale: WO 2012/010754

(56) Documents cités:
- WO-A1-2008/133681
- US-A- 4 498 978
- US-A1- 2009 133 577
- AUGIER F ET AL: "Liquid Drying by Solid Desiccant Materials: Experimental Study and Design Method", OIL & GAS SCIENCE AND TECHNOLOGY - REVUE DE L'INSTITUT FRANCAISDU PETROLE, EDITIONS TECHNIP, PARIS, FR, vol. 63, 1 novembre 2008 (2008-11-01), pages 713-722, XP009145165, ISSN: 0020-2274, DOI: DOI:10.2516/OGST:2008030 cité dans la demande
- "NEWTON'S CAVAILLON UNE AFFAIRE DE SPÉCIALISTES", DYNAMIQUES VAUCLUSE,, no. 44, 6 juillet 2006 (2006-07-06), pages 14-15, XP009145253,
- "Original industrial solutions for drying and neutralizing", NEWTON'S, CAVAILLON, FR, 3 décembre 2009 (2009-12-03), pages 1-6, XP009145311,
- "Newton's. Specialist in desiccant products", COMPANY BROCHURE, 24 novembre 2004 (2004-11-24), XP009145378,
- Newton's: "Briquettes: an optimal shape", , 7 juillet 2008 (2008-07-07), XP002625839, Extrait de l'Internet: URL:http://www.newton-s.com/our-products/b riquettes-an-optimized-shape/view?set_lang uage=en [extrait le 2011-03-02]

## Description

La présente invention concerne un procédé pour la déshydratation ou le séchage de liquides à l'aide d'agents déshydratants déliquescents sous forme de coussinets, de soude, autrement appelés solide alcalins. En particulier, l'invention a pour objet d'utiliser dans un procédé la saumure résultant du séchage ou de la déshydratation, ladite saumure étant appelée solution alcaline.

On connaît le document "Liquid Drying by Solid Desiccant Materials: Experimental Study and Design Method", F. Augier, C. Boyer and M. Vassieu, Oil & Gas Science and Technoloy - Rev. IFP, Vol. 63 (2008), No. 6, pp. 713-722, qui décrit le séchage par produits déliquescents.

On connaît l'utilisation de sels ou de saumure pour la déshydratation, mais la méthode et le dispositif selon la présente invention ne sont pas décrits.

Ainsi, la présente invention concerne un procédé de déshydratation d'un effluent liquide ou gazeux dans lequel on effectue les étapes suivantes:
- on neutralise ledit effluent par une saumure de soude, ,
- on déshydrate ledit effluent neutralisé sur des coussinets de soude,
- on collecte la saumure résultant de la déshydratation et on l'utilise dans ladite étape de neutralisation.

Selon le procédé, les coussinets peuvent avoir des dimensions comprises entre 1 et 3 cm.

Dans le procédé, l'effluent peut être un hydrocarbure, tel le LPG, une essence, un gasoil, un kérosène.

L'invention sera mieux comprise et ses avantages apparaîtront plus clairement à la lecture de la description qui suit d'exemples de réalisation, nullement limitatifs, illustrés par les figures ci-après annexées, parmi lesquelles:
- la figure 1 schématise le principe de l'invention,
- la figure 2 illustre un procédé d'adoucissement d'hydrocarbure par extraction des composés soufrés selon l'invention.

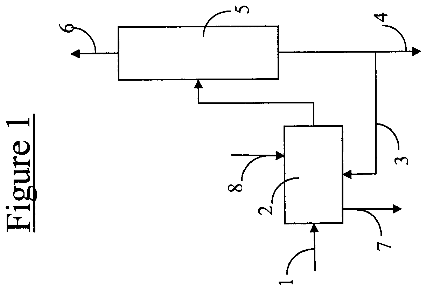

La figure 1 montre le principe général d'un procédé dans lequel un produit à neutraliser et à sécher entre par un conduit 1 dans des moyens de neutralisation 2 où l'on utilise une solution alcaline pour réaliser la neutralisation, ou adoucissement, de composés présents dans le produit. Le produit ainsi traité entre dans une colonne de séchage 5 remplie de solides alcalins, ou produits déliquescents, de même nature chimique que la solution alcaline utilisée dans les moyens 2. En contact avec les solides alcalins, le produit à traiter se déshydrate en formant une solution alcaline s'écoulant en fond de la colonne de séchage 5 via le conduit 4. Le produit traité sort de la colonne 5 par un conduit en tête 6. Une partie ou la totalité de la solution alcaline sortant en fond de colonne 5 est recyclée dans les moyens d'adoucissement 2 via le conduit 3. Ce recyclage n'exclut pas un appoint de solution alcaline dans le ballon 2 via un conduit 8. Une purge 7 de solution alcaline est également possible. L'intérêt d'utiliser dans la colonne de séchage un solide de même nature que la solution alcaline utilisée dans le procédé de neutralisation permet de limiter l'alimentation en solution alcaline neuve tout en diminuant l'effluent sortant en pied de colonne de séchage.

De préférence, on utilise des produits commercialisés par la société Newton's (France) sous la forme de grains de soude (NaOH). Les formes particulières en coussinets présentent un comportement optimum des grains au fur et à mesure de la consommation du produit.

La forme en coussinet a une surface spécifique propre plus grande que celle d'une perle de même poids ou de même volume, tout en limitant les pertes de surfaces liées aux contacts entre grains comme c'est le cas avec des formes en écailles. Elle est un optimum de la surface spécifique réellement disponible.

La forme en coussinets permet le réarrangement du lit au fur et à mesure que celui-ci est consommé. Il y a une diminution progressive de la hauteur du lit jusqu'à atteindre la hauteur minimum de lit requise par le procédé de déshydratation.

Les sels déliquescents absorbent l'humidité et se dissolvent dans l'eau absorbée pour former une saumure de mélange de sel et d'eau.

Les saumures générées par la déshydratation sont donc des saumures de soude à des concentrations comprises entre 20 et 60% massique.

Les procédés d'extraction de mercaptans par une solution de soude sont particulièrement pertinent dans le cadre de l'invention, car ils permettent d'en tirer un grand bénéfice. Ces procédés sont couramment utilisés en raffinerie pour extraire certains composés soufrés, par exemples des mercaptans, d'hydrocarbures variés comme par exemple le GPL, essence, gasoil, kérosène. Il existe un grand nombre de variantes de ce type de procédés, suivant le type de produit à traiter et la qualité du produit final désiré.

Les procédés en question comprennent généralement les opérations suivantes:
- une étape de prélavage à la soude diluée (entre 1 et 10%) destinée à la neutralisation des acides, par exemple H2S, en entrée de procédé,
- une extraction à contre-courant à la soude diluée (entre 10 et 20%), comprenant une faible quantité de catalyseur (non actif dans cette étape), les mercaptans (RSH) sont alors dissociés en mercaptides (RS-),
- une étape d'oxydation à l'air des mercaptides en disulfures, dans laquelle le catalyseur présent dans le soude est actif,
- une étape d'extraction des disulfures par un hydrocarbure, suivie d'une décantation en 1 ou 2 étapes,
- le recyclage de la soude régénérée en tête de colonne d'extraction liquide-liquide.

Une fois traitée, la charge est généralement lavée à l'eau. La charge lavée passe ensuite sur un filtre à sable dans le but de la séparer de l'eau libre. La teneur résiduelle d'eau dans la charge est selon les filtres de l'art antérieur comprise entre 50 et 100 ppm.

Selon l'invention, on remplace le filtre à sable par un ballon de séchage contenant un solide déliquescent composé de pastilles solides de soude. Ceci présente plusieurs avantages :
- La déshydratation réalisée est alors plus poussée, car elle ne se limite pas à l'eau libre. On peut obtenir une teneur en eau finale entre 10 et 50 ppm suivant la nature des charges.
- Les pastilles de soude utilisées génèrent une saumure qui est récupérée en pied de colonne de séchage. Cette saumure peut être diluée et utilisée: soit dans la colonne de prélavage à la soude, soit comme appoint dans la boucle d'extraction. Ceci présente un avantage important par rapport à l'utilisation de filtres à sables, car dans ce dernier cas l'eau libre extraite ne se présente pas sous forme de saumure, et doit être obligatoirement traitée. En utilisant des pastilles de soude, la saumure concentrée récupérée en pied de colonne de séchage peut éventuellement être réutilisée à l'extérieur du procédé si un flux à neutraliser existe par exemple sur le site industriel, mais son recyclage au sein même du procédé est bien plus intéressant. En effet, le recyclage de la saumure dans le procédé la produisant permet:- 1) de ne produire de la saumure que lorsque le procédé est en fonctionnement, ce qui permet d'éviter l'usage de bacs de stockages inhérents à une valorisation externe, - 2) la saumure est produite à proximité géographique de son usage, d'où des économies de conduites et de pompes.
- Le passage de la charge dans un lit fortement concentré en soude (pure) peut permettre de fixer les traces d'H2S voire de mercaptans encore présentes dans celle-ci.

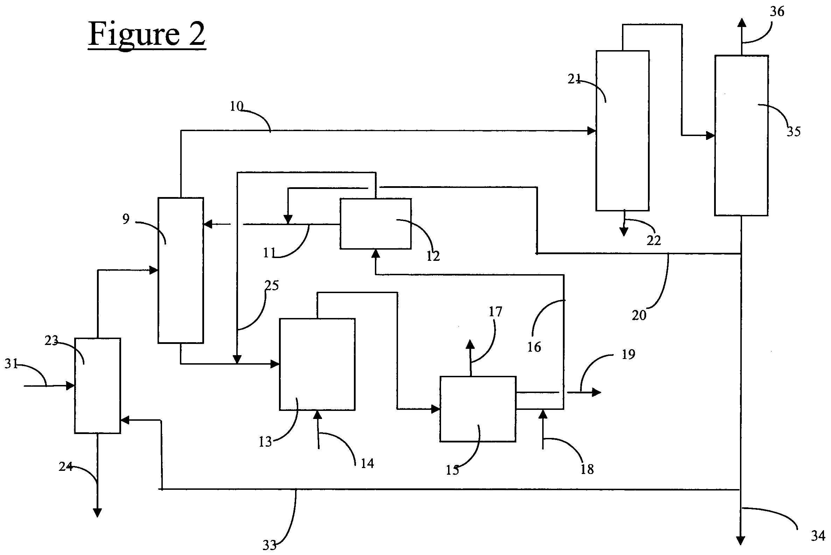

La figure 2 donne un exemple de procédé d'extraction de mercaptans présents dans un hydrocarbure, mettant en oeuvre l'invention. La charge ou produit à traiter, qui contient entre autres de l'H2S et des mercaptans entre via le conduit 31 dans une colonne de prélavage 23 contenant de la soude diluée. Cette colonne 23 sert à éliminer la majorité de l'H2S présent dans la charge, et élimine également au moins une petite partie des mercaptans. L'effluent sortant par le conduit 24 contient de la soude diluée, de l'H2S, des mercaptans et d'autres acides présents initialement dans la charge. Le produit sortant en tête de colonne 23 entre ensuite dans une colonne d'extraction liquide-liquide 9. La colonne d'extraction 9 est également alimentée par une solution de soude via le conduit 11, diluée entre 10 et 20% massique. L'extraction est généralement opérée en contre courant, la charge hydrocarbure monte et la solution de soude descend. La grande majorité des mercaptans est alors extraite dans la phase soude, sous forme de mercaptides. La solution de soude chargée en mercaptides est alors acheminée vers un réacteur d'oxydation 13 des mercaptides en disulfures. L'oxydation est réalisée à l'aide d'une injection d'air 14. L'oxydation est réalisée par catalyse en phase homogène. Le catalyseur utilisé préférentiellement est de la famille des phtalocyanines, et est dissous en phase soude. Le mélange polyphasique sortant du réacteur 13 entre dans un ballon de séparation 15. Le ballon 15 est utilisé pour séparer la phase gazeuse, qui en sort par le conduit 17, la phase soude via le conduit 16, et une phase organique via le conduit 19 contenant les disulfures. Le procédé se base sur le caractère fortement hydrophobe des disulfures qui sont très facilement extraits par une phase hydrocarbure. C'est pourquoi un hydrocarbure, par exemple une essence, est injectée dans le circuit de soude 16 via le conduit 18. Cet hydrocarbure extrait les disulfures présents dans la soude. Les deux phases soude et essence sont séparées dans un ballon de décantation 12. La soude ainsi régénérée est renvoyée vers la colonne d'extraction 9 via le conduit 11. L'essence sort du ballon de décantation 12 par le conduit 25 et est mélangée à la soude sortant de la colonne d'extraction 9. L'essence traverse ensuite le réacteur d'oxydation 13 et extrait ensuite les disulfures qui y sont formés. L'injection de l'essence fraîche en sortie de ballon 15 permet de finir l'extraction des disulfures et ainsi limiter le retour de soufre dans le colonne d'extraction.

Le produit désoufré sortant de la colonne d'extraction est acheminé par le conduit 10 vers une colonne de lavage à l'eau 21. Il sort de cette colonne un effluent aqueux en pied de colonne 22. Le produit lavé passe ensuite dans une colonne de séchage 35. Dans le cadre de l'invention, cette colonne est remplie de soude solide. Le produit déshydraté sort de la colonne 35 via la conduite 36. La colonne de séchage produit une solution de soude qui sort de la colonne 35 via le conduit 34, et dont la concentration est supérieure à celle utilisée dans la colonne de prélavage 23 ou dans la colonne d'extraction 9. Une fraction ou la totalité de la solution de soude produite lors de la déshydratation peut être recyclée dans le procédé : soit dans la colonne de prélavage via le conduit 33, soit en n'importe quel point de la boucle de solution de soude passant par la colonne d'extraction 9, le réacteur d'oxydation 13 et les ballons de séparation 15 ou 12. Sur la figure 2 le recyclage d'une partie de solution de soude produite dans la colonne de séchage 35 est recyclé via le conduit 20 dans le conduit 11 juste à l'entrée de la colonne d'extraction 9. La solution de soude peut être diluée à l'eau avant injection pour être utilisée à la concentration la plus pertinente.

Une mise en oeuvre pertinente de l'invention consiste à recycler la saumure dans la colonne de prélavage 23 uniquement lorsque la charge hydrocarbure est très concentrée en mercaptans. Il n'est pas rare de voir fluctuer la concentration en mercaptans dans l'hydrocarbure à traiter, donc la mise en marche du recyclage de la saumure quand la charge à traiter est concentrée permet de stabiliser le fonctionnement global du procédé et ainsi d'améliorer ses performances.

Une autre mise en oeuvre intéressante consiste à recycler en permanence la saumure dans la colonne de prélavage 23. Ainsi, par rapport au fonctionnement sans recyclage, la consommation de saumure est identique mais les performances du procédé sont améliorées du fait d'un prélavage fonctionnant en moyenne à une concentration en soude plus importante. Les espèces soufrées telles que mercaptans, COS ou H₂S sont ainsi plus efficacement captées par la phase aqueuse durant le prélavage.

Les avantages de l'invention peuvent donc être : - soit d'économiser de la soude, - soit d'augmenter les performances globales du procédé global de désulfuration.

## Revendications

1. Procédé de déshydratation d'une charge hydrocarbonée (31) contenant des mercaptans et de l'H2S dans lequel on effectue les étapes suivantes:
a) un prélavage de ladite charge (31) à la soude diluée entre 1 et 10% dans une colonne de prélavage (23),
b) une extraction liquide-liquide (9) à la soude (33) diluée entre 10 et 20% massique de l'effluent sortant en tête de colonne de prélavage (23) selon l'étape a) produisant une solution de soude chargée en mercaptides et un produit désoufré (10),
c) mélange de ladite solution de soude chargée en mercaptides avec un hydrocarbure (25),
d) une oxydation (13) dudit mélange issu de l'étape c) à l'aide d'une injection d'air (14) par catalyse en phase homogène, produisant un mélange polyphasique contenant des disulfures,
e) une séparation (15) de ladite solution polyphasique contenant des disulfures en une phase gazeuse (17), une phase aqueuse contenant la soude (16) et une phase organique contenant les disulfures (19),
f) une extraction desdits disulfures contenus dans la solution aqueuse (16) contenant la soude obtenue lors de l'étape e) par un hydrocarbure (18) pour obtenir un mélange contenant de la soude dépourvue de disulfures et ledit hydrocarbure,
g) une décantation (12) dudit mélange issu de l'étape f) permettant la récupération de soude régénérée dépourvue de disulfures d'une part (11), et dudit hydrocarbure d'autre part (25)
h) un recyclage (11) de ladite soude régénérée issue de l'étape g) vers la colonne d'extraction liquide-liquide (9) de l'étape b),
i) un lavage, dans une colonne de lavage à l'eau (21), du produit désoufré obtenu à l'étape b) permettant l'obtention d'un effluent lavé,
j) un séchage de l'effluent lavé issu de l'étape i) dans une colonne de séchage (35) remplie de soude solide sous forme de coussinets, permettant l'obtention du produit déshydraté d'une part (36) et d'une solution de soude d'autre part,
k) une collecte de ladite solution de soude résultant de l'étape j) et un recyclage, partiel ou total, de ladite solution vers l'étape b) d'extraction liquide-liquide.

2. Procédé selon la revendication 1, dans lequel lors de l'étape k) le recyclage, partiel ou total, de ladite solution a lieu vers l'étape b) d'extraction liquide-liquide et vers l'étape a) de prélavage (33).

3. Procédé selon l'une des revendications 1 à 2, dans lequel ledit effluent est un hydrocarbure, tel le LPG, une essence, un gasoil, un kérosène.

4. Procédé selon l'une des revendications 1 à 3, dans lequel lesdits coussinets ont des dimensions comprises entre 1 et 3 cm.

## Patentansprüche

1. Verfahren zur Dehydratisierung einer Kohlenwasserstoffcharge (31), die Mercaptane und H2S enthält, wobei die folgenden Schritte durchgeführt werden:
a) eine Vorwäsche der Charge (31) mit Natriumhydroxid, das zwischen 1 und 10 % verdünnt ist, in einer Vorwaschsäule (23),
b) eine Flüssig-Flüssig-Extraktion (9) mit Natriumhydroxid (33), das zwischen 10 und 20 Gew.-% verdünnt ist, des Abflusses, der am Kopf der Vorwaschsäule (23) gemäß Schritt (a) austritt, der eine Natriumhydroxidlösung erzeugt, die mit Mercaptiden beladen ist, und ein entschwefeltes Produkt (10),
c) die Mischung der mit Mercaptiden beladenen Natriumhydroxidlösung mit einem Kohlenwasserstoff (25),
d) eine Oxidation (13) der Mischung aus Schritt c) mit Säure aus einer Lufteinspritzung (14) durch Katalyse in homogener Phase, die ein mehrphasiges Gemisch, enthaltend Disulfide, erzeugt,
e) eine Trennung (15) der mehrphasigen Lösung, die Disulfide enthält, in eine gasförmige Phase (17), eine wässrige Phase, die Natriumhydroxid (16) enthält, und eine organische Phase, die Disulfide (19) enthält,
f) eine Extraktion der Disulfide, die in der in Schritt e) erhaltenen wässrigen Lösung (16), die das Natriumhydroxid enthält, enthalten sind, durch einen Kohlenwasserstoff (18), um ein Gemisch zu erhalten, das ein disulfidfreies Natriumhydroxid und den Kohlenwasserstoff enthält.
g) eine Dekantierung (12) des Gemischs aus Schritt f), die einerseits die Gewinnung von regeneriertem disulfidfreiem Natriumhydroxid (11) und andererseits des Kohlenwasserstoffs (25) ermöglicht,
h) eine Rückführung (11) des regenerierten Natriumhydroxids aus Schritt g) in die Flüssig-Flüssig-Extraktionssäule (9) aus Schritt b),
i) eine Wäsche, in einer Wasserwaschsäule (21), des in Schritt b) erhaltenen entschwefelten Produkts, die den Erhalt eines gewaschenen Abflusses ermöglicht,
j) eine Trocknung des gewaschenen Abflusses aus Schritt i) in einer Trocknungssäule (35), die mit festem Natriumhydroxid in Form von Kissen gefüllt ist, die einerseits den Erhalt des dehydratisierten Produkts (36) und andererseits einer Natriumhydroxidlösung ermöglicht,
k) eine Sammlung der aus Schritt j) resultierenden Natriumhydroxidlösung und eine teilweise oder vollständige Rückführung der Lösung in Schritt b) zur Flüssig-Flüssig-Extraktion.

2. Verfahren nach Anspruch 1, wobei im Verlauf von Schritt k) die teilweise oder vollständige Rückführung der Lösung in Schritt b) zur Flüssig-Flüssig-Extraktion und in Schritt a) zur Vorwäsche (33) stattfindet.

3. Verfahren nach einem der Ansprüche 1 bis 2, wobei der Abfluss ein Kohlenwasserstoff ist, wie beispielsweise LPG, ein Benzin, ein Gasöl, ein Kerosin.

4. Verfahren nach einem der Ansprüche 1 bis 3, wobei die Kissen Abmessungen im Bereich zwischen 1 und 3 cm umfassen.

## Claims

1. A process for the dehydration of a hydrocarbon feed (31) containing mercaptans and H₂S, in which the following steps are carried out:
a) prewashing of said feed (31) with caustic soda diluted to between 1% and 10% in a prewash column (23),
b) liquid-liquid extraction (9) of the effluent leaving the head of the prewash column (23) of step a) using caustic soda (33) diluted to between 10% and 20% by weight, producing a solution of caustic soda charged with mercaptides, and a desulphurized product (10),
c) mixing said solution of caustic soda charged with mercaptides with a hydrocarbon (25),
d) oxidizing (13) said mixture obtained from step c) with the aid of an injection of air (14) by homogeneous phase catalysis, producing a polyphase mixture containing disulphides,
e) separating (15) said polyphase solution containing disulphides into a gas phase (17), an aqueous phase containing caustic soda (16) and an organic phase containing the disulphides (19),
f) extracting said disulphides contained in the aqueous solution (16) containing caustic soda obtained during step e) with a hydrocarbon (18) in order to obtain a mixture containing caustic soda which is free from disulphides, and said hydrocarbon,
g) decanting (12) said mixture obtained from step f) in order to recover regenerated caustic soda which is free from disulphides on the one hand (11), and from said hydrocarbon on the other hand (25),
h) recycling (11) said regenerated caustic soda obtained from step g) to the liquid-liquid extraction column (9) of step b),
i) washing the desulphurized product obtained in step b) in a water scrubbing column (21) in order to obtain a scrubbed effluent,
j) drying the scrubbed effluent obtained from step i) in a drying column (35) filled with solid caustic soda in the form of pellets in order to obtain a dehydrated product on the one hand (36) and a solution of caustic soda on the other hand,
k) collecting said caustic soda solution resulting from step j) and recycling part or all of said solution to the liquid-liquid extraction step b).

2. The process as claimed in claim 1, in which during step k), the partial or complete recycling of said solution is carried out to the liquid-liquid extraction step b) and to the prewashing step a), (33).

3. The process as claimed in claim 1 or claim 2, in which said effluent is a hydrocarbon such as LPG, a gasoline, a gas oil or a kerosene.

4. The process as claimed in one of claims 1 to 3, in which said pellets have dimensions in the range 1 to 3 cm.
